# FASCICULE DE BREVET EUROPEEN

(11) **EP 0 491 023 B1**
(45) Date de publication et mention de la délivrance du brevet: **12.02.1997**
(21) Numéro de dépôt: 91912485.9
(22) Date de dépôt: 04.07.1991
(51) Int. Cl.: C12Q 1/18, C12Q 1/04

(54) **Procédé microbiologique de détection de produits toxiques à l'aide de souches de levures**
Mikrobiologisches Verfahren zum Nachweis toxischen Produkten mit Hilfe von Hefestämmen
Microbiological process for detecting toxic products by means of yeast strains

(30) Priorité: 05.07.1990 FR 9008552
(43) Date de publication de la demande: 24.06.1992
(73) Titulaire: COMMISSARIAT A L'ENERGIE ATOMIQUE, 75015 Paris (FR)
(72) Inventeur: JACOB, François, F-69200 Venitieux (FR); PERRIER, Joseph, F-69004 Lyon (FR); MOLEGNANA, Jacques, F-69100 Villeurbanne (FR); FAUGERAS, Pierre, F-30130 Pont-S.-Esprit (FR)
(74) Mandataire: Des Termes, Monique
(86) Numéro de dépôt international: FR9100544
(87) Numéro de publication internationale: WO9201064

(56) Documents cités:
- FR-A- 2 444 078
- BIOLOGICAL ABSTRACTS, vol. 84, no. 7, 1987, (Philadelphia, PA, US), H. TANAKA et al.:, "A new immobilized cell system with protection against toxic solvents", voir page AB-377, colonne 1, abrégé no. 65891, Biotechnol. Bioeng. 30(1), 1987, 22-30 (cité dans la demande)

## Description

La présente invention concerne un procédé microbiologique de détection de produits toxiques, minéraux ou organiques, et les souches utilisables pour la mise en oeuvre de ce procédé.

Elle s'applique en particulier au contrôle des eaux de distribution, des effluents et autres déchets industriels, agricoles ou urbains, destinés à être traités par voie biologique.

Les rejets de substances toxiques, permanents ou accidentels par certaines industries, entreprises agricoles, centres hospitaliers etc. constituent une source de contamination susceptible de perturber plus ou moins gravement des écosystèmes naturels ou des procédés industriels de traitement biologique des déchets.

En effet, les substances toxiques telles que les métaux lourds (plomb, mercure, sélénium, cadmium, cuivre, zinc, chrome, nickel, etc.), et les composés organiques tels que les cyanures, les composés organohalogénés, etc., se révèlent très nocifs pour l'environnement et représentent des produits particulièrement dangereux, soit par toxicité directe, soit par bioaccumulation dans des organismes maillons des chaînes alimentaires. Aussi, la présence de ces produits toxiques dans des liquides tels que les eaux de distribution, les effluents et autres, doit être détectée le plus rapidement possible.

De même, la présence de ces substances toxiques doit être détectée dans les déchets industriels qui vont être soumis à des procédés de traitement par voie biologique, par exemple fermentation microbienne aérobie ou anaérobie, car la plupart des microorganismes sont sensibles à ces produits toxiques, qui nuisent au bon déroulement du traitement par voie biologique.

En effet, comme il est indiqué par H. TANAKA et al dans "A new immobilized cell system with protection against toxic solvents", & BIOTECHNOL. BIOENG. 30(1) : 22-30, 1987, la fermentation éthanolique de microorganismes du type Saccharomyces cerevisiae, est inhibée par des solvants organiques tels que le 2-octanol, le benzène, le toluène et le phénol. On peut toutefois protéger ces microorganismes contre l'action de tels solvants, en les immobilisant dans un gel d'alginate auquel des huiles végétales telles que l'huile de ricin, l'huile d'olive ou l'huile de soja, ont été ajoutées.

On connaît certains procédés de détection de produits toxiques dans un milieu liquide ; en particulier, on peut mesurer les phénomènes d'inhibition engendrés par ces produits toxiques, par exemple par l'intermédiaire des variations de l'activité d'une enzyme spécifique ou sur la quantité de métabolite produite par un organisme vivant, comme il est décrit par Walker dans Fate and Effects of Pollutants, June 1989, p. 1077-1097, et par Munawar dans Hydrobiologia 149:87-105 (1987).

Ces procédés connus ne sont toutefois pas utilisables dans tous les cas et ne permettent pas forcément l'obtention d'une réponse rapide.

La présente invention a précisément pour objet un procédé microbiologique de détection de produits toxiques qui permet, d'une part, une détection rapide des produits toxiques présents dans un milieu plus ou moins complexe et, d'autre part, l'estimation de leur forme biologiquement active.

Selon l'invention, le procédé de détection d'un produit toxique présent dans un milieu consiste à cultiver des microorganismes sensibles à ce produit toxique et capables de réaliser une fermentation éthanolique, dans un milieu de culture convenant pour cette fermentation et contenant un échantillon dudit milieu, et à déterminer le taux d'inhibition de fermentation éthanolique provoqué par la présence du produit toxique dans cet échantillon, lesdits microorganismes étant des levures capables de produire au moins 1,5ml de CO₂/h par mg de biomasse, et de donner lieu à une baisse significative du dégagement de CO₂ en présence du produit toxique à détecter après au plus 1 heure de contact avec ce produit.

En effet, les levures sont des capteurs biologiques qui ont la particularité d'être proches des organismes vivants supérieurs car eucaryotes.

Les levures utilisées dans le procédé de l'invention possèdent ainsi les propriétés suivantes :
1°) une bonne productivité de CO₂ lors de la fermentation car il est nécessaire qu'une quantité suffisante de gaz soit dégagée pour que la mesure puisse être significative, et
2°) une bonne sensibilité au produit toxique, car l'on peut observer rapidement une baisse significative du dégagement gazeux lors d'un contact avec le produit toxique.

Par ailleurs, il est important que la levure ne modifie pas le produit toxique à détecter, par exemple ne réduise pas de manière conséquente sa solubilité dans le milieu comme c'est le cas par exemple avec certaines levures dégageant du H₂S qui précipitent les métaux lourds et réduisent de ce fait la toxicité du milieu.

Enfin, il est nécessaire de choisir des levures permettant d'obtenir une bonne reproductibilité des résultats.

De telles levures peuvent appartenir au genre Saccharomyces.

De préférence, on utilise la souche Saccharomyces LyA1 déposée sous le numéro I-964 le 27/6/90 à la Collection Nationale de cultures de Microorganismes de L'Institut Pasteur, que l'on désignera ci-après sous la référence LyA1, ou la souche Saccharomyces LyB1 déposée sous le numéro I-965 le 27/6/90 à la Collection Nationale de Cultures de Microorganismes de L'Institut Pasteur, que l'on désignera ci-après sous la référence LyB1.

Les caractéristiques des souches LyA1 et LyB1 sont données dans les tableaux 1 et 2 annexés.

On peut toutefois utiliser dans le procédé de l'invention d'autres microorganismes capables de réaliser une fermentation éthanolique à condition qu'ils aient une bonne sensibilité à des doses minimes du produit toxique à détecter, c'est-à-dire qu'une dose minime de produit toxique entraîne une inhibition détectable de la fermentation éthanolique.

Selon l'invention, le taux d'inhibition de fermentation éthanolique peut être déterminé en mesurant, soit la quantité de gaz carbonique produite par fermentation, soit la quantité de métabolite produite, par exemple d'éthanol.

Ainsi, on peut déterminer le taux d'inhibition de fermentation éthanolique à partir des mesures des quantités de CO₂ ou d'éthanol produites par fermentation dans les mêmes conditions, en présence et en l'absence du produit toxique à détecter.

Les conditions de fermentation sont choisies en fonction du microorganisme utilisé. Dans le cas où ce microorganisme est une levure du genre Saccharomyces, on réalise de préférence la fermentation éthanolique dans un milieu de culture ayant un pH compris entre 2,5 et 7, avantageusement à pH 4, en atmosphère exempte d'oxygène. De préférence, encore on réalise la fermentation à une température de 18 à 36°C, avantageusement à 28°C.

L'échantillon de milieu à analyser qui contient le produit toxique à détecter peut être soumis préalablement à un prétraitement de nature physique ou physicochimique, avant d'être utilisé dans le milieu de culture pour la fermentation. Ce prétraitement peut consister en une dilution, une concentration, une précipitation, ou une minéralisation. On peut utiliser aussi d'autres techniques séparatives telles que la chromatographie, les séparations sur résines, gels filtrants, et la centrifugation.

Ce prétraitement peut être effectué pour éliminer certains éléments gênants pour le procédé de détection par fermentation ou pour la détection et la mise en évidence de produits toxiques susceptibles d'être présents simultanément dans l'échantillon.

Ainsi, dans le cas où les produits toxiques à détecter sont constitués par des métaux lourds, on peut effectuer une séparation préalable de certains métaux présents dans l'échantillon, effectuer des mesures sur un échantillon non soumis au prétraitement et sur l'échantillon prétraité pour déterminer chacun des métaux lourds présents dans l'échantillon.

On peut réaliser également une détermination différentielle des différents produits toxiques en effectuant une première mesure à un premier pH du milieu, puis une seconde mesure après avoir modifié le pH car les profils d'inhibition peuvent être différents en fonction du pH et des produits toxiques à détecter.

Dans ce cas, on détermine les variations du taux d'inhibition en fonction du temps pour identifier le produit toxique présent dans l'échantillon.

Ainsi, le procédé de l'invention permet d'effectuer un diagnostic différentiel puis d'identifier certains toxiques métalliques, par exemple par l'examen du profil de cinétique d'inhibition.

Le procédé de l'invention peut aussi être utilisé pour détecter le seuil de concentration d'un produit toxique à ne pas dépasser. En effet, en choisissant une souche capable de réagir à un produit toxique à sa concentration de toxicité dans un milieu, on peut réaliser une sonde biologique jouant le rôle d'alarme.

On peut encore utiliser le procédé de l'invention pour déterminer la quantité et la nature du produit toxique, en choisissant une souche ayant une réponse différenciée selon la nature du produit toxique.

Le procédé de l'invention est très avantageux car il est facile à mettre en oeuvre et permet d'évaluer avec précision le degré de toxicité des produits toxiques biologiquement actifs présents.

Par rapport aux procédés connus, il se révèle très fiable et facile à utiliser en routine, même dans le cas de milieux liquides très colorés ou très chargés en particules, tels que des boues de stations d'épuration. Il peut d'ailleurs être utilisé pour la détection de produits toxiques dans des milieux plus ou moins complexes (opaques, liquides, pâteux ou hétérogènes).

Les produits toxiques susceptibles d'être détectés par le procédé de l'invention sont en particulier les métaux lourds tels que le plomb, le mercure, le cadmium, le sélénium, le cuivre, le zinc, le chrome, le nickel, etc. et leurs composés inorganiques ou organométalliques et les composés organiques et organohalogénés tels que le phénol, le naphtalène, les dérivés chlorés du benzène comme le phényl chlorobenzène, etc.

L'invention a également pour objet les microorganismes utilisés dans ce procédé de détection.

D'autres caractéristiques et avantages de l'invention apparaîtront mieux à la lecture de la description qui suit, donnée bien entendu à titre illustratif et non limitatif, en référence au dessin annexé sur lequel :
- la figure 1 est une représentation schématique d'un appareil utilisable pour mettre en oeuvre le procédé de l'invention,
- la figure 2 est un diagramme représentant l'inhibition (exprimée en pourcentage de l'activité fermentaire éthanolique) de la souche Ly B₁ en fonction de la concentration en mercure d'un échantillon,
- la figure 3 est un diagramme représentant l'inhibition (exprimée en µl de CO₂/hxmg de biomasse et en pourcentage de l'activité fermentaire éthanolique) de la levure Ly A₁ en fonction de la concentration en plomb d'un échantillon, et
- la figure 4 est un diagramme illustrant l'inhibition (exprimée en pourcentage de l'activité de fermentation) de la souche Ly B₁ en fonction du temps, soit en présence de plomb, (courbe en trait plein), soit en présence de mercure, (courbe en pointillés) après un changement de pH du milieu de fermentation de 6,0 à 4,0.

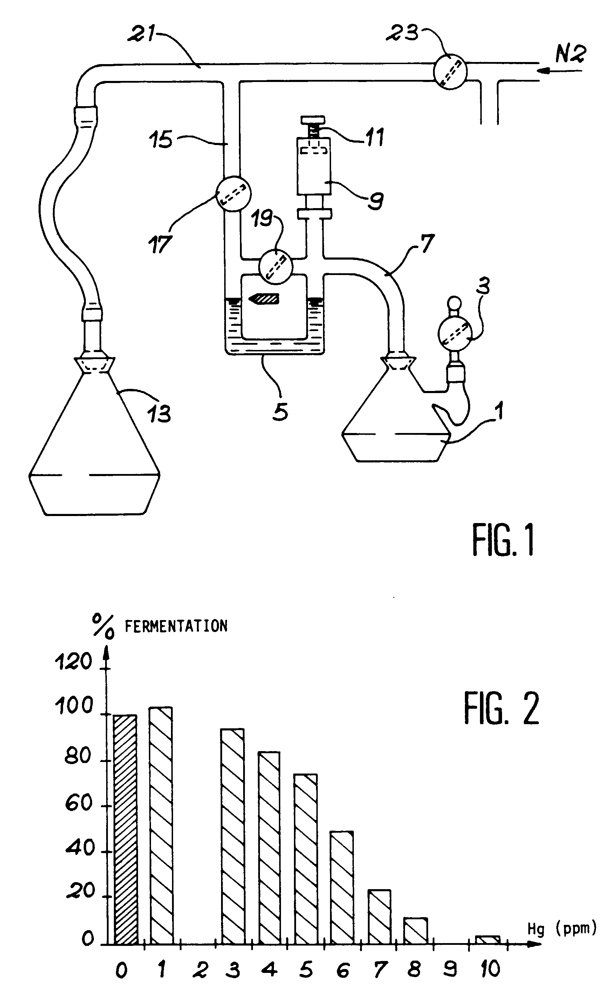

Sur la figure 1, on a représenté une installation pouvant être utilisée pour déterminer le taux d'inhibition de fermentation éthanolique par mesure de la quantité de gaz carbonique dégagé.

Ce dispositif comprend une fiole (1) contenant le milieu de culture et le microorganisme utilisé. On peut introduire dans cette fiole (1) par la vanne (3) l'échantillon du liquide à analyser. La fiole (1) est en communication par sa partie supérieure avec un tube en U (5) servant de manomètre par l'intermédiaire d'une conduite (7). Le gaz dégagé dans la fiole (1) peut être mesuré par le microvolumètre (9) étalonné en microlitres et muni d'une molette de réglage (11) pour maintenir une pression constante dans la fiole (1). L'autre branche du manomètre est reliée à une fiole (13) destinée à compenser des microvariations de pression extérieures au système. Les deux branches du manomètres peuvent être reliées entre elles par une conduite munie d'une vanne (19).

La fiole (13) et la conduite (15) débouchent dans une conduite générale (21)munie d'une vanne (23).

Pour mettre en oeuvre le procédé de l'invention, on introduit dans la fiole (1) dont le volume peut être d'environ 15ml, 2ml de milieu de culture qui renferme environ 10⁶ cellules du microorganisme utilisé et qui contient un volume variable de l'échantillon à analyser. On purge tout d'abord le microréacteur de son air par balayage sous flux de gaz inerte tel que l'azote, en ouvrant les vannes (17, 19 et 3) pendant 3 min et en introduisant ce gaz par la vanne (23) à un débit de 3l/min. On ferme ensuite successivement les vannes (23, 3 et 19), puis on détermine la variation de pression dans la conduite (7) due au dégagement de gaz carbonique en la compensant par une variation de la capacité du microvolumètre (9) en déplaçant la molette de réglage (11).

Les exemples suivants illustrent le dosage du mercure et du plomb par le procédé de détection de l'invention.

### Exemple 1 : Dosage du mercure utilisant la souche de levure Saccharomyces LyB1.

On utilise l'appareillage représenté sur la figure 1 et on introduit dans la fiole (1) 2ml du milieu de culture MFL et environ 2.10⁸ cellules de levure Saccharomyces L_{y}B₁.

Le milieu de culture MFL contient 20g/l de sucre fermentescible, 5g/l d'extrait de levure, et de l'eau distillée ou l'échantillon aqueux à analyser ou un mélange des deux.

On réalise plusieurs dosages en utilisant des milieux de culture MFL contaminés par 1 à 10ppm de mercure sous forme de HgCl₂.

Après avoir purgé l'ensemble de l'appareillage de son air par balayage d'azote pendant 3min à un débit de 3l/min, on réalise la fermentation à une température de 28°C et un pH de 4,0, en soumettant le milieu de culture à une agitation à raison de 60 translations par minute. Après 10min de fermentation, on mesure le volume de gaz carbonique dégagé dans le microvolumètre (9). La mesure de référence pour déterminer le taux d'inhibition est effectuée dans les mêmes conditions sans adjonction de HgCl₂, et elle correspond ici à 1100µl de CO₂ par heure et par milligramme de biomasse poids sec de levure.

Les résultats obtenus sont donnés sur la figure 2 qui représente l'inhibition (en % de l'activité fermentaire) par rapport à la quantité de mercure (en ppm) présente dans l'échantillon.

Sur cette figure, on voit que le seuil significatif de toxicité qui correspond au taux d'inhibition détectable, c'est-à-dire plus de 5% d'inhibition, est obtenu avec une concentration en mercure totale de l'échantillon de 3ppm, ce qui correspond à 40ppb (parties par billion) de mercure sous forme soluble.

Le procédé de détection de l'invention est donc très intéressant puisqu'il permet de détecter rapidement des quantités biologiquement actives très faibles de mercure.

### Exemple 2 : Dosage du plomb dans un milieu filtré de digestat au moyen de la souche de levure Saccharomyces LyA1.

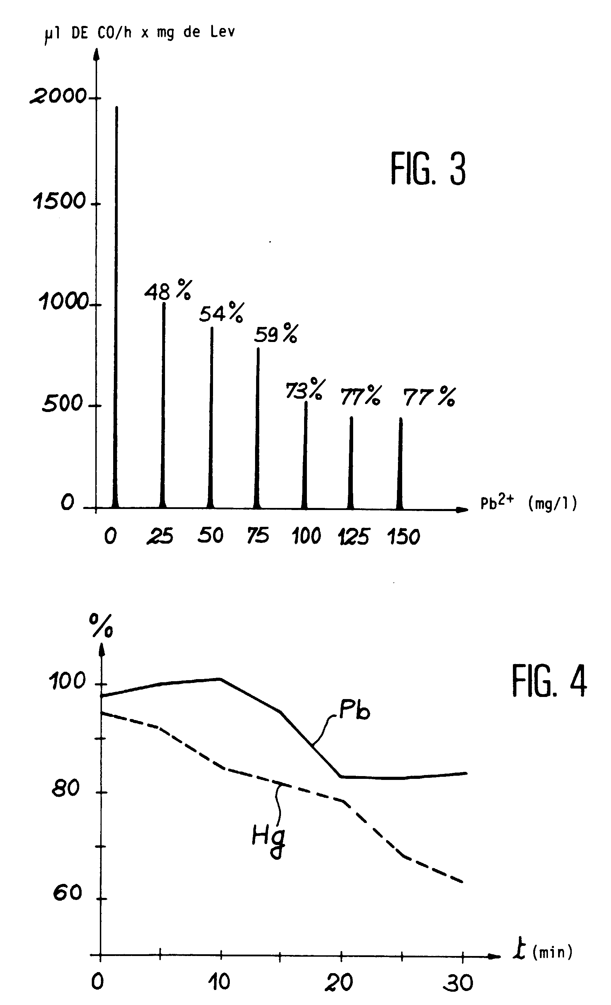

On suit le même mode opératoire que dans l'exemple 1 pour déterminer l'inhibition de fermentation éthanolique, mais dans ce cas on utilise la souche LyA1 et on réalise la culture à une température de 36°C et à un pH de 4, après avoir soumis au préalable l'échantillon à analyser à une filtration sur une membrane poreuse de 0,45µm. On détermine comme précédemment le taux d'inhibition en mesurant les quantités de CO₂ dégagées dans le microvolumètre (9). Les résultats obtenus sont donnés sur la figure 3 qui représente le volume de CO₂ dégagé par h par mg de poids sec de biomasse (levure) en fonction de la concentration totale en plomb (en mg/l). On a également précisé sur cette figure les taux d'inhibition correspondants (en %).

Ainsi, on voit qu'une concentration totale en plomb de 23mg/l correspond à un taux d'inhibition de 48%.

### Exemple 3 : Dosage du plomb et du mercure utilisant la souche LyB1.

On suit le même mode opératoire que dans l'exemple 1, pour réaliser les dosages sur des milieux de culture contenant respectivement 50mg/l de Pb et 8mg/l de Hg, sauf que l'on réalise la fermentation à un pH de 6 et que l'on modifie le pH pour l'ajuster à 4 après 30min de fermentation, sans modifier les autres conditions opératoires.

On observe alors des cinétiques d'inhibition différentes pour le plomb et le mercure.

Sur la figure 4, on a représenté l'inhibition (en % de l'activité fermentaire) en fonction du temps (en minutes), la courbe en trait plein se rapporte au milieu contenant 50mg/l de plomb et la courbe en pointillés se rapporte au milieu contenant 8mg/l de mercure.

Ainsi, en effectuant des mesures sur un milieu contenant du Pb ou du Hg avant et après le changement de pH, on peut suivant le profil d'inhibition obtenu, identifier le toxique présent dans le milieu.

Le procédé de l'invention est donc très intéressant puisqu'il permet d'établir un diagnostic différentiel des produits toxiques tels que les métaux lourds.

On peut aussi obtenir ce diagnostic différentiel en effectuant un prétraitement de séparation des métaux, par exemple sur résines échangeuses d'ions.

De plus, il peut être mis en oeuvre de façon automatique pour effectuer la surveillance séquentielle ou continue d'un milieu tel qu'un cours d'eau, un affluent ou un effluent d'installation industrielle telle qu'une station d'épuration, en utilisant par exemple une batterie de réacteurs alimentés en milieu de culture, en microorganismes et en échantillons à analyser.

**TABLEAU 1**

| Souche: *Saccharomyces* LyAl | | | |
|---|---|---|---|
| **ASSIMILATION** | | | **FERMENTATION** |
| **Substrat** | **3 jours** | **3 semaines** | |
| | | | |
| Glucose | + | + | + |
| Galactose | + | + | + |
| Saccharose | + | + | + |
| Maltose | + | + | + |
| Cellobiose | - | - | - |
| Trehalose | + | + | - |
| Lactose | - | - | - |
| Raffinose | + | + | + |
| Amidon sol. | - | - | |
| Xylose | - | - | **TEST COMPLEMENTS** |
| L-Arabinose | - | - | |
| Ribose | - | - | Croissance à 37°C: + |
| Rhamnose | - | - | |
| Erythritol | - | - | Ethylamine HCl: - |
| Ribitol | - | - | Cadavérine: - |
| Mannitol | - | - | |
| Ac succinique | - | - | Croissance 50% glucose: + |
| Ac citrique | - | - | Croissance 60% glucose: - |
| Inositol | - | - | |
| Ethanol | + | + | 0,1% cycloheximide: - |
| Glycerol | - | - | 0,01% cycloheximide: - |
| Nitrate | - | - | |
| Nitrite | - | - | **SPORULATION** |
| Sans vitamine | - | - | Fowel: + |
| Sorbose | - | - | |
| Melibiose | - | - | **LAMES GELOSEES** |
| Melezitose | + | + | Pseudomycélium: + |
| Inuline | - | + | vrai mycélium: - |
| D-Arabinose | - | - | |
| Glucosamine | - | - | |
| Sorbitol | - | - | |
| -methyl glucosidde | + | + | |
| Salicine | - | - | |
| Ac lactique | - | - | |
| Arbutine | - | - | |
| Dulcitol | - | - | |
| Xylitol | - | - | |

**TABLEAU 2**

| Souche: *Saccharomyces* LyBl | | | |
|---|---|---|---|
| **ASSIMILATION** | | | **FERMENTATION** |
| **Substrat** | **3 jours** | **3 semaines** | |
| | | | |
| Glucose | + | + | + |
| Galactose | + | + | + |
| Saccharose | - | - | - |
| Maltose | - | - | - |
| Cellobiose | - | - | - |
| Trehalose | - | - | - |
| Lactose | - | - | - |
| Raffinose | - | - | - |
| Amidon sol. | - | - | |
| Xylose | - | - | **TEST COMPLEMENTS** |
| L-Arabinose | - | - | |
| Ribose | - | + | Croissance à 37°C: + |
| Rhamnose | - | - | |
| Erythritol | - | - | Ethylamine HCl: - |
| Ribitol | - | - | Cadavérine: - |
| Mannitol | - | - | |
| Ac succinique | - | - | Croissance 50% glucose: - |
| Ac citrique | - | - | Croissance 60% glucose: - |
| Inositol | - | - | |
| Ethanol | +/- | + | 0,1% cycloheximide: - |
| Glycerol | - | - | 0,01% cycloheximide: + |
| Nitrate | - | - | |
| Nitrite | - | - | **SPORULATION** |
| Sans vitamine | - | - | Fowel: + |
| Sorbose | - | - | |
| Melibiose | - | - | **LAMES GELOSEES** |
| Melezitose | - | - | Pseudomycélium: - |
| Inuline | - | - | vrai mycélium: - |
| D-Arabinose | - | - | |
| Glucosamine | - | - | |
| Sorbitol | - | - | |
| -methyl glucosidde | - | - | |
| Salicine | - | - | |
| Ac lactique | - | - | |
| Arbutine | - | - | |
| Dulcitol | - | - | |
| Xylitol | - | - | |

## Revendications

1. Procédé de détection d'un produit toxique présent dans un milieu, caractérisé en ce qu'il consiste à cultiver des microorganismes sensibles à ce produit toxique et capables de réaliser une fermentation éthanolique, dans un milieu de culture convenant pour cette fermentation et contenant un échantillon dudit milieu, et à déterminer le taux d'inhibition de fermentation éthanolique provoqué par la présence du produit toxique dans cet échantillon, lesdits microorganismes étant des levures capables de produire au moins 1,5ml de CO₂/h par mg de biomasse, et de donner lieu à une baisse significative du dégagement de CO₂ en présence du produit toxique à détecter après au plus 1 heure de contact avec ce produit.

2. Procédé selon la revendication 1, caractérisé en ce que les microorganismes sont des levures appartenant au genre Saccharomyces.

3. Procédé selon la revendication 2, caractérisé en ce que la levure est la souche Saccharomyces LyA1 déposée sous le n° I-964 le 27/6/90 à la Collection Nationale de Cultures de Microorganismes tenue par l'Institut Pasteur.

4. Procédé selon la revendication 2, caractérisé en ce que la levure est la souche Saccharomyces LyB1 déposée sous le n° I-965 le 27/6/90 à la Collection Nationale de Culture de Microorganismes tenue par l'Institut Pasteur.

5. Procédé selon l'une quelconque des revendications 1 à 4, caractérisé en ce que l'on détermine le taux d'inhibition de fermentation éthanolique en mesurant les quantités de CO₂ produites par fermentation dans les mêmes conditions, en présence et en l'absence de l'échantillon contenant le produit toxique à détecter.

6. Procédé selon l'une quelconque des revendications 1 à 4, caractérisé en ce que l'on détermine le taux d'inhibition de fermentation éthanolique en mesurant les quantités d'éthanol produites par fermentation dans les mêmes conditions, en présence et en l'absence dudit produit toxique.

7. Procédé selon l'une quelconque des revendications 1 à 6, caractérisé en ce que le produit toxique est un métal lourd, un composé de métal lourd ou une molécule appartenant au groupe des toxiques organiques ou organohalogénés.

8. Procédé selon l'une quelconque des revendications 1 à 7, caractérisé en ce que l'on détermine les variations du taux d'inhibition en fonction du temps pour identifier le produit toxique présent dans l'échantillon.

9. Procédé selon la revendication 7, caractérisé en ce que le métal lourd est le plomb, le mercure, le cadmium, le sélénium, le cuivre, le zinc, le chrome, ou le nickel.

10. Procédé selon l'une quelconque des revendications 1 à 9, caractérisé en ce que l'on réalise la fermentation éthanolique dans un milieu de culture ayant un pH entre 2,5 et 7.

11. Procédé selon l'une quelconque des revendication 1 à 10, caractérisé en ce que l'on réalise la fermentation éthanolique à une température de 18 à 36°C.

12. Procédé selon l'une quelconque des revendications 1 à 11, caractérisé en ce que l'on réalise préalablement un prétraitement de l'échantillon contenant le toxique.

13. Procédé selon l'une quelconque des revendications 1 à 12, caractérisé en ce que le liquide est un effluent aqueux ou un déchet industriel, agricole ou urbain.

14. Souche de Saccharomyces LyA1 déposée sous le n° I-964 à la Collection Nationale de Cultures de Microorganismes de L'Institut Pasteur.

15. Souche de Saccharomyces LyB1 déposée sous le n° I-965 à la Collection Nationale de Cultures de Microorganismes de l'Institut Pasteur.

## Patentansprüche

1. Verfahren zum Nachweis eines toxischen Stoffes in einer Umgebung, dadurch gekennzeichnet, daß es die folgenden Schritte enthält:
Kultivierung von Mikroorganismen, die auf diesen toxischen Stoff sensibel reagieren und fähig sind, eine EthanolFermentation durchzuführen in einem für diese Fermentation geeigneten Kulturmedium, das eine Probe dieser Umgebung enthält und Bestimmung der Inhibitionsrate der Ethanolfermentation, ausgelöst durch die Gegenwart des toxischen Stoffs in dieser Probe, wobei die Mikroorganismen Hefen sind, die fähig sind, mindestens 1,5 ml CO₂/h pro mg Biomasse zu produzieren und ein signifikantes Abfallen des Freiwerdens von CO₂ in Gegenwart des toxischen Stoffes auszulösen, was nach mehr als 1 Stunde des Kontakts mit diesem Stoff festzustellen ist.

2. Verfahren gemäß Anspruch 1, dadurch gekennzeichnet, daß die Mikroorganismen Hefen der Gattung Saccharomyces sind.

3. Verfahren gemäß Anspruch 2, dadurch gekennzeichnet, daß die Hefe der Stamm Saccharomyces LyA1 ist, deponiert unter der Nr. I-964 am 27.06.1990 bei der Collection Nationale de Cultures de Microorganismes des Institut Pasteur.

4. Verfahren nach Anspruch 2, dadurch gekennzeichnet, daß die Hefe der Stamm Saccharomyces LyB1 ist, deponiert unter der Nummer I-965 am 27.06.1990 bei der Collection Nationale de Culture de Microorganismes des Institut Pasteur.

5. Verfahren nach einem oder mehreren der Ansprüche 1 - 4, dadurch gekennzeichnet, daß man die Inhibitionsrate der Ethanolfermentation bestimmt, indem man die durch Fermentation produzierten CO₂-Mengen unter denselben Bedingungen mißt, und zwar in Gegenwart und in Abwesenheit der Probe, die den zu bestimmenden toxischen Stoff enthält.

6. Verfahren gemäß einem oder mehreren der Ansprüche 1 - 4, dadurch gekennzeichnet, daß die Inhibitionsrate der Ethanolfermentation bestimmt wird, indem man die durch die Fermentation produzierten Ethanolmengen unter denselben Bedingungen mißt, in Gegenwart und in Abwesenheit des toxischen Stoffes.

7. Verfahren gemäß einem oder mehreren der Ansprüche 1 - 6, dadurch gekennzeichnet, daß der toxische Stoff ein Schwermetall ist, eine Verbindung des Schwermetalls oder ein Molekül aus der Gruppe der organischen oder organohalogenen toxischen Stoffe.

8. Verfahren gemäß einem oder mehreren der Ansprüche 1 - 7, dadurch gekennzeichnet, daß man die Änderungen der Inhibitionsrate als Funktion der Zeit bestimmt, um den toxischen Stoff, der in der Probe vorhanden ist, zu identifizieren.

9. Verfahren gemäß Anspruch 7, dadurch gekennzeichnet, daß das Schwermetall Blei ist, Quecksilber, Cadmium, Selen, Kupfer, Zink, Chrom oder Nickel.

10. Verfahren gemäß einem oder mehreren der Ansprüche 1 - 9, dadurch gekennzeichnet, daß man die Ethanolfermentation in einem Kulturmedium mit einem pH zwischen 2,5 und 7 realisiert.

11. Verfahren gemäß einem oder mehreren der Ansprüche 1 - 10, dadurch gekennzeichnet, daß man die Ethanolfermentation bei einer Temperatur von 18 bis 36 °C realisiert.

12. Verfahren gemäß einem oder mehreren der Ansprüche 1 - 11, dadurch gekennzeichnet, daß man die Probe, die den toxischen Stoff enthält, zunächst vorbehandelt.

13. Verfahren gemäß einem oder mehreren der Ansprüche 1 -12, dadurch gekennzeichnet, daß die Flüssigkeit ein Abwasser ist oder ein Industrieabfall, landwirtschaftlicher oder städtischer Abfall.

14. Saccharomyces Stamm LyA1, deponiert unter der Nummer I-964 bei der Collection Nationale de Culture des Microorganismes im Institut Pasteur.

15. Saccharomyces Stamm LyB1, deponiert unter der Nummer I-965 bei der Collection Nationale de Culture de Microorganismes im Institut Pasteur.

## Claims

1. Process for the detection of a toxic product present in a medium, characterized in that it consists of culturing microorganisms sensitive to said toxic product and able to perform an ethanolic fermentation in a culture medium suitable for said fermentation and containing a sample of said medium, and determining the ethanolic fermentation inhibition rate caused by the presence of the toxic product in said sample, said microorganisms being yeasts able to produce at least 1.5 ml of CO₂/h/mg of biomass and give rise to a significant reduction in CO₂ formation in the presence of the toxic product to be detected after at the most 1 hour contact with said product.

2. Process according to claim 1, characterized in that the microorganisms are yeasts of the Saccharomyces type.

3. Process according to claim 2, characterized in that the yeast is the Saccharomyces LyA1 strain deposited under No. I-964 on 27.6.1990 with the National Collection of Microorganism Cultures held by the Pasteur Institute.

4. Process according to claim 2, characterized in that the yeast is the Saccharomyces LyB1 strain deposited under No. I-965 on 27.6.1990 with the National Collection of Microorganisms Cultures held by the Pasteur Institute.

5. Process according to any one of the claims 1 to 4, characterized in that the ethanolic fermentation inhibition rate is determined by measuring the CO₂ quantities produced by fermentation under the same conditions, in the presence and absence of the sample containing the toxic product to be detected.

6. Process according to any one of the claims 1 to 4, characterized in that the ethanolic fermentation inhibition rate is determined by measuring the ethanol quantities produced by fermentation under the same conditions, in the presence and absence of said toxic product.

7. Process according to any one of the claims 1 to 6, characterized in that the toxic product is a heavy metal, a heavy metal compound or a molecule belonging to the group of organohalogen or organic toxic substances.

8. Process according to any one of the claims 1 to 7, characterized in that the variations of the inhibition rate are determined as a function of time for identifying the toxic product present in the sample.

9. Process according to claim 7, characterized in that the heavy metal is lead, mercury, cadmium, selenium, copper, zinc, chromium or nickel.

10. Process according to any one of the claims 1 to 9, characterized in that ethanolic fermentation takes place in a culture medium with a pH between 2.5 and 7.

11. Process according to any one of the claims 1 to 10, characterized in that ethanolic fermentation takes place at a temperature of 18 to 36°C.

12. Process according to any one of the claims 1 to 11, characterized in that the sample containing the toxic substance undergoes a pretreatment beforehand.

13. Process according to any one of the claims 1 to 12, characterized in that the liquid is an aqueous effluent or an industrial, agricultural or urban waste.

14. Saccharomyces strain LyA1 deposited under No. I-964 in the National Collection of Microorganism Cultures of the Pasteur Institute.

15. Saccharomyces strain LyB1 deposited under No. I-965 in the National Collection of Microorganism Cultures of the Pasteur Institute.
